# EUROPEAN PATENT SPECIFICATION

(11) **EP 3 793 529 B1**
(45) Date of publication and mention of the grant of the patent: **02.12.2026**
(21) Application number: 19723819.9
(22) Date of filing: 17.05.2019
(51) Int. Cl.: A61K 9/20, A61K 31/00

(54) **STABLE PHARMACEUTICAL COMPOSITIONS OF DPP-IV INHIBITORS IN COMBINATION WITH METFORMIN IN THE FORM OF IMMEDIATE RELEASE TABLETS**
STABILE PHARMAZEUTISCHE ZUSAMMENSETZUNGEN VON DPP-IV-INHIBITOREN IN KOMBINATION MIT METFORMIN IN FORM VON TABLETTEN MIT SOFORTIGER FREISETZUNG
COMPOSITIONS PHARMACEUTIQUES STABLES D'INHIBITEURS DE DPP-IV EN COMBINAISON AVEC DE LA METFORMINE SOUS LA FORME DE COMPRIMÉS À LIBÉRATION IMMÉDIATE

(30) Priority: 18.05.2018 EP 18382343
(43) Date of publication of application: 24.03.2021
(73) Proprietor: Galenicum Health S.L.U., 08950 Esplugues de Llobregat (ES)
(72) Inventor: TORREJON NIETO, Javier, 08005 Barcelona (ES); GOMEZ COELLO, Luis, 08005 Barcelona (ES)
(74) Representative: Galenicum Health S.L.U.
(86) International application number: PCT/EP2019/062826
(87) International publication number: WO 2019/219920

(56) References cited:
- WO-A1-2015/097234
- WO-A2-2006/078593
- CN-A- 106 551 927

## Description

The present invention relates to stable pharmaceutical compositions of vildagliptin, a DPP-IV inhibitor, in combination with metformin or pharmaceutically acceptable salts thereof. More specifically, the invention relates to compositions comprising a granulated portion comprising metformin and binder and a second portion comprising vildagliptin, and excipients in the form of immediate release tablets as well as to a process of manufacturing the pharmaceutical compositions.

### STATE OF THE ART

Type 2 diabetes is a chronic and progressive disease arising from a complex pathophysiology involving the dual endocrine defects of insulin resistance and impaired insulin secretion. The treatment of type 2 diabetes typically begins with diet and exercise, followed by oral antidiabetic monotherapy. For many patients, these regimens do not sufficiently control glycemia during long-term treatment, leading to a requirement for combination therapy within several years following diagnosis.

Dipeptidyl dipeptidase-IV (DPP-IV), also known as adenosine deaminase complexing protein 2 or CD26, is an antigenic enzyme expressed on the surface of most cell types and is associated with immune regulation, signal transduction and apoptosis. DPP-IV plays a major role in glucose metabolism. It is responsible for the
degradation of incretins such as GLP-1. A new class of oral hypoglycemics called DPP-IV inhibitors work by inhibiting the action of this enzyme, thereby prolonging incretin effect in vivo. They can be used to treat diabetes mellitus type 2. Glucagon increases blood glucose levels, and DPP-IV inhibitors reduce glucagon and blood glucose levels. The mechanism of DPP-IV inhibitors is to increase incretin levels
(GLP-1 and GIP), which inhibit glucagon release, which in turn increases insulin secretion, decreases gastric emptying, and decreases blood glucose levels.

Vildagliptin is a DDP-IV inhibitor also known as (2S)-1-{2-[(3-hydroxy-1-adamantyl)amino]acetyl}pyrrolidine-2-carbonitrile. The empirical formula of vildagliptin is C₁₇H₂₅N₃O₂ and the compound has a molecular weight of 303.4. The structural formula of vildagliptin is (I):

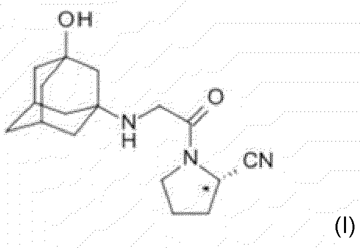

Metformin is used as a front line treatment in the management of type 2 diabetes. It lowers blood glucose preventing the production of glucose by the liver. Dipeptidyl peptidase IV (DPP-IV) inhibitors, also used in the treatment of diabetes, have a complementary mechanism of action. They primarily work by lowering the levels of incretin hormones which results in an increase in insulin and a reduction in glucagon levels. Because they work on different biochemical pathways, DPP-IV inhibitors can be safely administered alongside metformin. Combined tablets containing metformin and a DPP-IV inhibitor such as vildagliptin are now widely prescribed.

Metformin is also known as N,N-dimethylimidodicarbonimidic diamide. The empirical formula of metformin is C₄H₁₁N₅ and the compound has a molecular weight of 129.2 g/mol. Metformin is most often used as its hydrochloride salt. The structural formula of metformin HCl is (II):

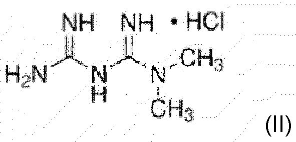

EP1948149 discloses tablets comprising vildagliptin and metformin manufactured by granulation of metformin using various methods such as wet, dry and melt granulation with a binder followed by compression of the metformin granules with vildagliptin and magnesium stearate. As stated by J. Lakshman and J. Kowalski during the opposition proceedings on June 4, 2013, the compositions of the above patent, when prepared by wet granulation using water, resulted in unacceptable friability, low hardness and sensitivity to humidity. Granulation with organic solvents also resulted in clumping of granules which made them difficult to tablet as well as tablets having longer disintegration times than desired. It was thus concluded that only compositions comprising vildagliptin and metformin prepared by melt granulation were suitable for industrial use. The marketed product, Eucreas^{®} comprise film coated tablets manufactured using melt granulation, however the melt granulation process has some disadvantages as it is expensive and requires specialised equipment.

EP3086781 discloses the manufacture of vildagliptin metformin tablets by wet granulation of the metformin with a binder such as hydroxypropylcellulose (HPC) and a diluent and subsequent compression with vildagliptin, and magnesium stearate as a powder or as granule. The presence of a diluent in the granules is reported as necessary to provide the necessary flowability, compression and compactness. It reports compositions comprising metformin and vildagliptin can be manufactured with a wet granulation step, however these compositions still have shortcomings, in particular with long disintegration times, between 10-40 min.

CN 106551927 discloses pharmaceutical compositions comprising vildagliptin and metformin hydrochloride prepared by a two-step process involving fluid-bed granulation of metformin alone to overcome its poor compressibility. Despite improving flowability, the resulting compositions require prolonged dissolution times and lack optimization regarding disintegration velocity and long-term physical stability.

It is an aim of the present invention to overcome the deficiencies and difficulties outlined above, particularly to provide tablets with better stability, shorter disintegration time and shorter dissolution profiles as well as process of manufacturing such tablets. It is a further aim to provide a tablet with good uniformity.

### SUMMARY OF THE INVENTION

The present invention provides stable pharmaceutical compositions of metformin and DPP-IV inhibitor such as vildagliptin in the form of immediate release tablets. The stable immediate release tablets as disclosed herein can be stored at temperatures higher than 25°C and are stable when packaged in aluminium/PVC.

Compositions according to the present invention are easy to manufacture and do not suffer from setbacks such as capping and sticking during manufacture. The pharmaceutical compositions have fast dissolution, low friability and high hardness. The compositions according to the present invention are also of an acceptable size for swallowing by a patient.

In a first aspect, the present invention relates to a pharmaceutical composition in the form of immediate release tablets comprising at least the following components defined by claims:
a) a granulated portion granulated prior to its incorporation into a tablet which comprises metformin or a pharmaceutically acceptable salt thereof, an amount of water below 5 wt%, relative to the weight of the granulated portion, and optionally a binder.
b) a second portion, comprises vildagliptin or a pharmaceutically acceptable salt thereof, wherein vildagliptin is crystalline vildagliptin, and wherein vildagliptin is not in the form of granules, a diluent, a disintegrating agent and a lubricant.

The diluent of the second portion is a mixture of lactose and microcrystalline cellulose, wherein the lactose is in an amount of 1 to 15% and the microcrystalline cellulose 1 to 5%, and the lubricant is selected from the group comprising magnesium stearate and sodium stearyl fumarate.

In a second aspect, the present invention relates to a method for the manufacture of stable pharmaceutical compositions of metformin and vildagliptin in the form of immediate release tablets, wherein the process comprises the following steps:
a) Granulating the metformin, preferably metformin hydrochloride, and optionally a binder, to form a granulated portion;
b) Blending the granulated portion with the second portion comprising vildagliptin, the diluent which is a mixture of lactose and microcrystalline cellulose, a disintegrating agent and a lubricant to obtain a final blend;
c) Compressing the final blend of step c) into tablets;
d) Optionally, applying a coating on the tablets of step c).

### DETAILED DESCRIPTION OF THE INVENTION

The present invention provides stable pharmaceutical compositions of metformin or pharmaceutically acceptable salts thereof combined with a DPP-IV inhibitor such as vildagliptin or pharmaceutically accepted salts thereof, wherein the composition comprises a granulated portion comprising metformin and a second portion comprising vildagliptin.

The terms "filler" and "diluent" are used interchangeably in this specification. "Diluent" as used herein refers to pharmaceutically acceptable excipients which are added to the bulk volume of the active agent making up the solid composition. As a result, the size of the solid composition increases, which makes its size suitable for handling. Diluents are convenient when the dose of drug per solid composition is low and the solid composition would otherwise be too small.

As used herein, "lubricant" means a substance that reduces friction between the composition of the present invention and the surfaces of the apparatus used to compact the composition into a compressed form.

As used herein, "binder" means a substance or a mixture of substances added to a tablet to facilitate the formation of granules for the granulated portion.

As used herein, "disintegrant" means a substance or a mixture of substances added to a tablet to facilitate its breakup or disintegration after administration. The terms "disintegrant" and "disintegrating agent" are used interchangeably in this specification.

The term "stable" as used herein refers to a pharmaceutical composition comprising metformin combined with a vildagliptin wherein the total content of impurities of the composition is below 5 % (w/w), preferably 3 % (w/w), more preferably 2 % (w/w), and most preferably 1 % (w/w), as determined by liquid chromatography (HPLC) with the detector set at 210 nm for vildagliptin and 252 nm for metformin if such a composition is stored for 2 months at 40°C and 75 % relative humidity (RH).

When the pharmaceutical composition as herein disclosed comprises excipients such as a filler, an acid, a lubricant or a disintegrant, these excipients are pharmaceutically acceptable. The term "pharmaceutically acceptable" indicates that the substance or composition must be compatible chemically and/or toxicologically, with the other ingredients comprising a formulation, and/or the mammal being treated therewith.

The term "active ingredient" refers to a therapeutically active compound, as well as any prodrugs thereof and pharmaceutically acceptable salts, hydrates and solvates of the compound and the prodrugs.

All percentages, parts, and ratios herein are by weight unless specifically noted otherwise. As used herein, the term "about" refers preferably to a range that is ±10 %, preferably ±5 %, or more preferably ±1 % of a value with which the term is associated.

Unless otherwise indicated, all the analysis methods are carried out according to Ph.Eur. 2.9.3. Dissolution Test for Solid Dosage Forms.

An immediate release tablet as herein disclosed has to be understood as a tablet having a dissolution performance such as 75% or more of the active agent contained in said pharmaceutical composition dissolves within 45 minutes (min). In a most preferred embodiment, the pharmaceutical composition as herein disclosed releases at least 60% of the active agent in 15 min, and further preferably at least 90% of the active agent in 30 min. The dissolution test for a pharmaceutical composition comprising the active agent as herein disclosed is performed according to Ph.Eur. 2.9.3. The relevant parameters of the Ph.Eur. 2.9.3 dissolution test for Solid Dosage Forms are described below:
(i) Dissolution Media pH 1.2: 0.1N HCl, paddle, 50 rpm, 1000mL, 37 ± 0.5 °C, or
(ii) Dissolution Media pH 6.8: Phosphate Buffer, paddle, 50 rpm, 1000mL, 37 ± 0.5 °C. Determination by HPLC: detector 210 nm for vildagliptin and 252 nm for metformin.

The immediate release tablets of the present invention herein disclosed, have a disintegration time of less than 30 minutes. The immediate release tablet of the present invention has a disintegration time of less than 20 minutes. Preferably the tablet has a disintegration time of less than 15 minutes. More preferably, the tablet has a disintegration time of less than 12 minutes.

The granulated portion may be formed by any conventional granulation method, for example wet granulation or dry granulation methods. Preferably, wet granulation methods are used for forming the granulated portion. Preferably, water is used for the wet granulation. Preferably, water is used for the wet granulation and also used as a binder, or water might be present in the excipient used in the composition, or present in pharmaceutical composition as absorbed from the environment.

The granulated portion comprises an amount of water below 5 wt%, preferably from 0.1 to 4 wt %, preferably 0.3 to 3 wt % and further preferably from 0.4 to 1.4 wt % relative to the weight of the granulated portion. This range has been found to provide improved hardness.

Preferably the granulated portion comprises from 80 to 100% metformin. More preferably, the granulated portion comprises from 80 to 99 % metformin. More preferably, the granulated portion comprises 82 to 98 % metformin when dry. More preferably, the granulated portion comprises 85 to 95 % metformin when dry. Preferably the granulated portion does not comprise any additional excipients other than the binder. Preferably the granulated portion does not comprise any additional excipients other than hydroxypropyl cellulose (HPC) as the desired properties are achieved by this. Preferably, the total amount of metformin present in the pharmaceutical composition ranges from 60 to 90% by weight in respect of the total amount of the pharmaceutical composition, more preferably 65 to 79%.

Preferably the total amount of metformin or salt thereof per tablet is from about 500 mg to about 1000 mg and the amount of vildagliptin or salt thereof per tablet is about 50 mg. Preferably, the total amount of metformin or salt thereof per tablet is of about 500 or 850 or 1000 mg and the amount of vildagliptin or salt thereof per tablet is about 50 mg.

The granulated portion also comprises an amount of water up to 5%, preferably from 0.1 to 4 wt %, preferably 0.3 to 3 wt % and further preferably from 0.4 to 1.4 wt % of the granulated portion. As a result of it, the pharmaceutical composition also comprises an amount of water up to 4.5 %, from about 0.08 to 4.2 wt %, preferably 0.25 to 2.8 wt %, further preferably from about 0.4 to 1.3 wt % of the total amount of the pharmaceutical composition and most preferably below or equal to 0.6 wt%. This has been found to improve the friability, hardness, stability and dissolution times of the resulting composition.

Preferably the granulated portion is passed through a sieve having openings of less than 5 mm prior to compression into a tablet, further preferably the openings are 3 mm and most preferably 2 mm or less. The size of the granules of the granulated portion have a D90 preferably from 250 µm up to 700 µm, more preferably from 300 µm to 580 µm. A granulated portion comprising granules of this size have been found to produce tablets with improved hardness and uniformity.

The granulated portion comprises a binder. In a preferred embodiment the binder may be selected from acacia, tragacanth, sucrose, sodium alginate, methyl cellulose, hydroxypropyl cellulose, pyrrolidone and ethyl cellulose. Preferably said binder is hydroxypropyl cellulose.

Preferably the binder has a particle size of less than 1 mm.

Preferably, the granulated portion comprises 0 to 15 % binder. More preferably, the granulated portion comprises 1 to 10 % binder. More preferably granulated portion comprises form 1 to 8 % In one embodiment, granulation of metformin may also be carried out using water as a binder. The amount of water in the final composition will be close to zero as the water may be evaporated during the manufacturing process.

Preferably, the total amount of binder present in the pharmaceutical composition ranges from 0 to 14 % by weight in respect of the total amount of the pharmaceutical composition. More preferably, the total amount of binder present in the pharmaceutical composition ranges from 0 to 9 % by weight in respect of the total amount of the pharmaceutical composition, and further preferably from 3 to 7 % by weight in respect of the total amount of the pharmaceutical composition.

The second portion comprises vildagliptin, which may be present as a powder. The powder may be very fine, fine, moderately coarse or coarse according to the USP standard terminology. Very fine powders of chemicals have a maximum diameter of ≤ 125 µm, fine ≤ 180 µm or moderately coarse ≤ 425 µm. It may be crystalline or amorphous. Preferably the second portion comprises vildagliptin or salts thereof, a diluent, a lubricant and a disintegrant. Preferably the second portion does not comprise any further excipients.

According to the invention, the second portion is not granulated.

The granulated portion and the second portion are combined by using a standard compression method to form tablets. Preferably they are combined after the granulated portion has been completely dried.

In a preferred embodiment, the tablets are coated. The tablets may be coated with any conventional coating agent. The coating agent may be a polymer based coating agent and preferably comprises a cellulose base polymer such as hypromellose (HPMC) or a polyvinyl alcohol based polymer, such as polyvinyl alcohol (PVA). Preferably the coating agent comprises titanium dioxide and talc. Such coating agents have been found to improve friability and hardness of the composition. Preferably the coating agent comprises PVA based polymer. Examples of suitable coating agents are those made under the trade name Opadry^{®}, for example Opadry 02F220039 Yellow and 02F220038 Yellow, Opadry^{®} II (HPMC containing) and Methocel^{®}. Preferably the coating comprises no more than 5% of the total mass of the pharmaceutical composition. Further preferably, the coating agent comprises from 1% to 3% of the total mass of the pharmaceutical composition. The coating agent may comprise colouring or flavouring compounds.

In a preferred embodiment of the pharmaceutical composition as herein disclosed, said pharmaceutical composition comprises from 1 to 10 % by weight of vildagliptin or a pharmaceutically acceptable salt thereof, in respect of the total amount of the pharmaceutical composition. Preferably, it comprises from 2 to 7 % by weight of vildagliptin or a pharmaceutically acceptable salt thereof, in respect of the total amount of the pharmaceutical composition. More preferably, it comprises from 2 to 6 % by weight of vildagliptin or a pharmaceutically acceptable salt thereof, in respect of the total amount of the pharmaceutical composition. Preferably vildagliptin is in the form of a free base. Vildagliptin is in crystalline form, preferably vildagliptin is in powder form. Vildagliptin is not in the form of granules. Most preferably, vildagliptin is a crystalline powder and further preferably it is in its free base form. Preferably, vildagliptin has a particle size volume distribution D90 from about 500 to 50 µm, more preferably a D90 from about 500 to 150 µm, further preferably from about 450 to 200 µm, and optimally a D90 from about 450 to 260 µm, as determined preferably by a laser particle size analyser, such as the Mastersizer^{®} range. This range has been found to provide better uniformity and flowability.

The term 'Dx' as used herein means that x% of the particles in a composition (based on volume) have a diameter of or below a specified d value. Thus, a D90 of 100 µm means that 90% of the particles, by volume, have a diameter of or below 100 µm. As well as using D90 as a measuring reference to determine particle size, d95 is occasionally used for such a purpose. Therefore, a D95 of 100 µm means that 95% of the particles, by volume, have a diameter of or below 100 µm.

Preferably the vildagliptin in crystalline form as form A which is described in the patent document WO2006/078593A2 (Novartis). Crystalline Form A is characterised by X-Ray diffraction peaks at 12.0, 13.5, 16.6, 17.1, 17.2, 20.1, 22.5, 27.4 and 28.1 ± 0.2 degrees 2-theta. Also crystalline Form A is characterised by FTIR, which shows the characteristic bands at about 3293 cm-1, 2925-2853 cm-1, 2238 cm-1, 1658 cm-1, 1455/1354 cm-1, 1254 cm-1, 1054-1035 cm-1, ±2 cm-1.

The diluent of the second portion is a mixture of microcrystalline cellulose and lactose.

The presence of microcrystalline cellulose and lactose provides a more stable pharmaceutical composition, particularly reducing the total percentage of impurities and particularly the vildagliptin derived impurities. Lactose anhydrous is preferable to lactose monohydrate in this formulation as is has been found to further reduce the concentration of impurities.

Preferably the total amount of diluent is from 2 to 16 %, preferably from 5 to 15 % and still preferably from 8 to 12% in respect of the total amount of the pharmaceutical composition.

The second portion comprises a diluent comprising mixture of lactose and microcrystalline cellulose. In a particularly preferred embodiment, the diluent in the second portion comprises from 5% to 11% lactose, preferably anhydrous lactose, in respect of the total amount of the pharmaceutical composition and form 1% to 5% microcrystalline cellulose in respect of the total amount of the pharmaceutical composition. Further preferably, the diluent of the second portion comprises 7% to 10% lactose, preferably anhydrous lactose, in respect of the total amount of the pharmaceutical composition and 1% to 4% microcrystalline cellulose in respect of the total amount of the pharmaceutical composition. It has been found that the addition of microcrystalline cellulose shortens the disintegration time of the tablet and improves stability. Microcrystalline cellulose has also been found to increase the rate of dissolution.

Preferably the diluent has a particle size of less than 1 mm, further preferably less than 0.813 mm. Preferably the diluent has been passed through a 1 mm sieve or further preferably through a 0.813 mm sieve. By "1 mm" sieve, a sieve having apertures of 1 mm is meant.

Preferably, the granulated portion, or granules, do not comprise lactose monohydrate or lactose anhydrous.

The granulated portion may comprise a lubricant. The second portion comprises a lubricant selected from magnesium stearate and sodium stearyl fumarate. Preferably, the total amount of lubricant or lubricants present in the pharmaceutical composition ranges from 0.1 to 5 % by weight in respect of the total amount of the pharmaceutical composition. More preferably, the total amount of lubricant present in the pharmaceutical composition ranges from 0.5 to 2 % by weight in respect of the total amount of the pharmaceutical composition.

The lubricant is in the second portion and the total amount of lubricant or lubricants present in the pharmaceutical composition ranges from 0.5 to 2 % by weight in respect of the total amount of the pharmaceutical composition.

In a preferred embodiment of the pharmaceutical composition as herein disclosed, said pharmaceutical composition comprises at least a lubricant selected from stearic acid, magnesium stearate, polyethylene glycol, sodium stearyl fumarate (SSF), sodium benzoate and mixtures thereof. Preferably, the pharmaceutical composition comprises at least a lubricant selected from magnesium stearate, stearic acid, sodium stearyl fumarate or mixtures thereof, more preferably the pharmaceutical composition comprises sodium stearyl fumarate. Sodium stearyl fumarate has been found to provide shorter disintegration times than other similar lubricants such as magnesium stearate. Sodium stearyl fumarate has also been found to increase the dissolution rate compared to magnesium stearate.

Preferably the lubricant has a particle size of less than 1 mm, preferably less than 0.5 mm, preferably less than 0.42 mm.

The pharmaceutical composition comprises a disintegrant. The disintegrant may be present in the granulated portion. The second portion comprises a disintegrant. Preferably, the granulated portion does not comprise a disintegrant. Preferably, the total amount of disintegrant or disintegrants present in the pharmaceutical composition ranges from 0.1 to 10% by weight in respect of the total amount of the pharmaceutical composition. More preferably, the total amount of disintegrant or disintegrants present in the pharmaceutical composition ranges from 0.5 to 3% by weight in respect of the total amount of the pharmaceutical composition.

In a preferred embodiment of the pharmaceutical composition as herein disclosed, said pharmaceutical composition comprises at least a disintegrant selected from watersoluble disintegrants, such as starch, pregelatinized starch, sodium carboxymethyl cellulose, croscarmellose sodium (CCS), crosslinked polyvinylpyrrolidone, low-substituted hydroxypropyl cellulose, sodium starch glycolate and mixtures thereof. Preferably, the pharmaceutical composition comprises at least a disintegrant selected from starch, pregelatinized starch, and low-substituted hydroxypropyl cellulose, croscarmellose sodium, more preferably the pharmaceutical composition comprises croscarmellose sodium.

Another embodiment of the present invention relates to a method for the manufacture of stable pharmaceutical compositions of metformin and vildagliptin in the form of immediate release tablets as defined in the claims, wherein the process comprises the following steps:
a) Granulating the metformin, and optionally a binder, to form a granulated portion;
b) Blending the granulated portion with the second portion comprising vildagliptin, the diluent, a disintegrating and a lubricant to obtain a final blend;
c) Compressing the final blend into tablets;
d) Optionally, applying a coating on the tablets obtained in step c).

In a preferred embodiment, the present invention relates to a method for the manufacture of stable pharmaceutical compositions of metformin and vildagliptin in the form of immediate release tablets as defined in the claims, wherein the process comprises the following steps:
a) Granulating the metformin, and optionally a binder, to form a granulated portion;
b) Blending the granulated portion with the second portion comprising vildagliptin, the diluent, a disintegrating and a lubricant to obtain a final blend;
c) Compressing the final blend into tablets;
d) Optionally, applying a coating on the tablets of step c);
wherein the granulated portion is granulated by wet granulation, preferably wherein the solvent for the wet granulation is water. The inventors have found that the amount of moisture has to be carefully controlled during the process in order to achieve the desired technical effects.

In a preferred embodiment, the tablets are packaged in an aluminium/aluminium blister pack. The term "blister" or bubble pack refers to a sheet in a hermetically sealed package construction with recesses designed to hold dosage forms.

A preferred embodiment of the present invention is a pharmaceutical composition in the form of immediate release tablets as defined in the claims comprising at least a granulated portion which was granulated prior to its incorporation into a tablet, and
at least a second portion,
wherein the granulated portion comprises metformin or a pharmaceutically acceptable salt thereof, an amount of water from 0.3 to 3 wt %, relative to the weight of the granulated portion, and optionally a binder, and
wherein the second portion comprises vildagliptin or a pharmaceutically acceptable salt thereof, the diluent, a disintegrating agent and a lubricant,
wherein the metformin is in the form of metformin hydrochloride and/or wherein vildagliptin in its free base form, wherein the total amount of metformin is from 60 to 90%,
wherein the granulated portion is formed by wet granulation, wherein the solvent for the wet granulation is water, wherein the lubricant is selected from the group comprising magnesium stearate and sodium stearyl fumarate, wherein the lubricant present in an amount from 0.1 to 5%.

Another preferred embodiment of the present invention is a pharmaceutical composition in the form of immediate release tablets as defined in the claims, the tablets comprising at least a granulated portion which was granulated prior to its incorporation into a tablet, and
at least a second portion,
wherein the granulated portion comprises metformin or a pharmaceutically acceptable salt thereof, an amount of water from 0.3 to 3 wt %, relative to the weight of the granulated portion, and optionally a binder, and
wherein the second portion comprises vildagliptin or a pharmaceutically acceptable salt thereof, a diluent, a disintegrating agent and a lubricant,
wherein the diluent is a mixture of lactose and microcrystalline cellulose, wherein the total amount of diluent is from 5 to 15 % in respect of the total amount of the pharmaceutical composition, and
wherein the composition comprises a coating agent, wherein the coating agent comprises a polyvinyl alcohol based polymer or hypromellose, and the coating agent is present in an amount of from 1 to 5%, in respect of the total amount of the pharmaceutical composition.

Another preferred embodiment of the present invention is a pharmaceutical composition in the form of immediate release tablets as defined in the claims, the tablets comprising at least a granulated portion which was granulated prior to its incorporation into a tablet, and
at least a second portion,
wherein the granulated portion comprises metformin or a pharmaceutically acceptable salt thereof, an amount of water from 0.3 to 3 wt %, relative to the weight of the granulated portion, and optionally a binder, and
wherein the second portion comprises vildagliptin or a pharmaceutically acceptable salt thereof, a diluent, a disintegrating agent and a lubricant,
wherein the metformin is in the form of metformin hydrochloride and/or wherein vildagliptin in its free base form, wherein the total amount of metformin is from 60 to 90%,
wherein the granulated portion is formed by wet granulation, wherein the solvent for the wet granulation is water, wherein the disintegrant is selected from a group comprising crospovidone, croscarmellose sodium, wherein the disintegrant is present in an amount of 0.2 to 5% by weight in respect of the total amount of the pharmaceutical composition.

A most preferred embodiment of the present disclosure is a pharmaceutical composition in the form of immediate release tablets as defined in the claims, the tablets comprising at least a granulated portion which was granulated prior to its incorporation into a tablet, and
at least a second portion,
wherein the granulated portion comprises metformin or a pharmaceutically acceptable salt thereof, an amount of water from 0.3 to 3 wt %, relative to the weight of the granulated portion, and optionally a binder, and
wherein the second portion comprises vildagliptin or a pharmaceutically acceptable salt thereof, a diluent, a disintegrating agent and a lubricant,
wherein the metformin is in the form of metformin hydrochloride and/or wherein vildagliptin in its free base form, wherein the total amount of metformin is from 60 to 90%, wherein the total amount of vildagliptin is from 2 to 7 % by weight, wherein the granulated portion is formed by wet granulation, wherein the solvent for the wet granulation is water, wherein the diluent is a mixture of lactose anhydrous and microcrystalline cellulose wherein the lactose is present in an amount of from 1 to 15% by weight, in respect of the total amount of the pharmaceutical composition and wherein the microcrystalline cellulose is preferably present in an amount of from 1 to 5% by weight, in respect of the total amount of the pharmaceutical composition, wherein the disintegrant is selected from a group comprising crospovidone, croscarmellose sodium, wherein the disintegrant is present in an amount of 0.2 to 5% by weight in respect of the total amount of the pharmaceutical composition, wherein the lubricant is selected from the group comprising magnesium stearate and sodium stearyl fumarate, wherein the lubricant present in an amount from 0.1 to 5%, wherein the water content of the film coated tablet measured with Karl Fischer titration is below 1.5 w%.

### EXAMPLES

### Examples 3, 4, 6, and 7 are not according to the claimed invention

**Table 1A**

| Examples 1 and 2: Combined metformin vildagliptin tablet composition | | | | |
|---|---|---|---|---|
| **Example No.** | **1** | | **2** | |
| | mg/tablet | % | mg/tablet | % |
| **Granulated portion** | | | | |
| Metformin hydrochloride | 850 | 75.76% | 1000 | 76.39% |
| Hydroxypropyl cellulose | 60 | 5.35% | 70 | 5.35% |
| Purified water | q.s. | | q.s. | |

| **Second portion** | | | | |
|---|---|---|---|---|
| Vildagliptin (free base) | 50 | 4.46% | 50 | 3.82% |
| Lactose anhydrous | 96 | 8.56% | 115 | 8.79% |
| Microcrystalline cellulose | 23 | 2.05% | 25 | 1.91% |
| Croscarmellose sodium | 10 | 0.89% | 15 | 1.15% |
| Sodium stearyl fumarate | 9 | 0.80% | 10 | 0.76% |

| **Coating** | | | | |
|---|---|---|---|---|
| Opadry^{®} Yellow | 24 | 2.14% | 24 | 1.83% |
| Total | 1122 | | 1309 | |

**Table 1B**

| Examples 3. 4 and 5: Combined metformin vildagliptin tablet composition | | | | | | |
|---|---|---|---|---|---|---|
| **Example No.** | **3** | | **4** | | **5** | |
| | mg/tablet | % | mg/tablet | % | mg/tablet | % |
| **Granulated portion** | | | | | | |
| Metformin HCl | 1000 | 77.4% | 1000 | 76.5% | 1000 | 76.5% |
| Hydroxypropylcellulose | 68 | 5.3% | 68 | 5.2 | 68 | 5.2% |
| Purified water | q.s. | | q.s. | | q.s | |

| **Second portion** | | | | | | |
|---|---|---|---|---|---|---|
| Vildagliptin (free base) | 50 | 5.9% | 50 | 3.8% | 50 | 3.8% |
| Lactose anhydrous | 120 | 9.3% | 134 | 10.3 | 105 | 8.0% |
| Microcrystalline cellulose | - | | - | | 28 | 2.1% |
| Sodium stearyl fumarate | - | | 11.25 | 0.9% | 12.8 | 1% |
| Magnesium stearate | 10 | 0.8% | - | | - | |
| Croscarmellose sodium | 13.5 | 1.1% | 13.5 | 1.0% | 13 | 1.0% |

| **Coating** | | | | | | |
|---|---|---|---|---|---|---|
| OpadryYellow | 30 | 2.3% | 30 | 2.3% | 30 | 2.3% |
| Total (mg/tablet) | 1291.5 | | 1306.8 | | 1306.8 | |

In Table 1B, the total mass of the tablet is experimentally determined.

Examples 1 - 5 were prepared according to the following process:
a) Granulating metformin hydrochloride, hydroxypropyl cellulose and water to form a granulated portion;
b) Blending the granulated portion with free base vildagliptin, lactose anhydrous, optionally microcrystalline cellulose, the croscarmellose sodium and the lubricant to obtain a final blend;
c) Compressing the final blend into tablets;
d) Applying Opadry^{®} Yellow coating on the tablets of step c).

Capping was not observed during the process for Examples 1-5. Good uniformity and disintegration times as well as acceptable friability were obtained.

**Table 2**

| Examples 6 and 7: Combined metformin vildaaliptin tablet composition | | | | |
|---|---|---|---|---|
| **Example No.** | **6** | | **7** | |
| | mg/tablet | % | mg/tablet | % |
| **Granulated portion** | | | | |
| Metformin HCl | 1000 | 74.5% | 1000 | 74.5% |
| Hydroxypropylcellulose | 68 | 5.06% | 68 | 5.06% |
| Microcrystalline cellulose | 75 | 5.6% | 37.5 | 2.8% |
| Fumed silica | 21 | 1.56% | 21 | 1.56% |
| Vildagliptin (free base) | 50 | 3.72% | 50 | 3.72% |

| **Second portion** | | | | |
|---|---|---|---|---|
| Microcrystalline cellulose | - | | - | |
| Lactose anhydrous | 75 | 5.58% | 112.5 | 8.38% |
| Magnesium stearate | 10 | 0.74% | 10 | 0.74% |
| Croscarmellose sodium | 13.5 | 1.01% | 13.5 | 1.01% |

| **Coating** | | | | |
|---|---|---|---|---|
| Opadry^{®} Yellow | 30.6 | 2.28% | 30.6 | 2.28% |
| Total (mg/tablet) | 1343 | | 1343 | |

Examples 6 and 7 were manufactured according to the following process:
a) Milling the metformin hydrochloride,
b) Sifting vildagliptin, hydroxypropyl cellulose, microcrystalline cellulose, and fumed silica and blending with the milled metformin hydrochloride in a blender,
c) Compacting the blended mixture using a roller compactor to produce flakes with a thickness of 2.25 to 2.55 mm,
d) Milling the flakes to produce the granulated portion,
e) Sifting the croscarmellose sodium, magnesium stearate and lactose anhydrous or microcrystalline cellulose and blending,
f) Adding blended mixture of step e) to granulated portion and compacting to produce a tablet.

### Stability

Tablets of examples 3, 4 and 5 were packaged in bottles or in blisters packs of aluminium/aluminium. Tablets of the marketed product, Eucreas^{®} 50mg/1000mg Film Coated Tablets and Examples 3, 4 and 5 of the present disclosure were tested at 25 °C and 60 RH. These studies are designed and conducted according to the European Medicines Agency *Guideline on stability testing of existing active substances and related finished products* (CPMP/QWP/122/02, rev 1 corr).

**Table 3. Total impurities with relative response factors; 25°C and 60% RH; Pack: (ALU/ALU Blister)**

| | Example 3 | Example 4 | Example 5 |
|---|---|---|---|
| 3 months | 0.45 | 0.48 | 0.25 |

From the above table, it can be seen that after three months at 25°C and 60% RH, tablets prepared according to the invention show low levels of impurities. Tablets prepared according to Example 5 comprising microcrystalline cellulose and sodium stearyl fumarate show a lower level of impurities than Examples 3 and 4. This is an unexpected advantage of extragranular microcrystalline cellulose in the second portion.

### Dissolution

All the analysis methods are carried out according to Ph.Eur. 2.9.3. Dissolution Test for Solid Dosage Forms. Determination by HPLC: 210 nm for vildagliptin and 252 nm for metformin.

**Table 4A Dissolution Media: pH 1.2: 0.1N HCl, paddle, 50 rpm, 1000mL**

| | Example 3 | | Example 5 | |
|---|---|---|---|---|
| minutes | Metformin | Vildagliptin | Metformin | Vildagliptin |
| 15 | 67 | 66 | 79 | 72 |
| 30 | 100 | 102 | 97 | 97 |

From the above table it can be seen that at the 15 minutes mark, that in the formulations according to the disclosure at least 60% of the active ingredient is released by 15 minutes and at least 95% is released by 30 minutes in acidic conditions. The release, or dissolution, of the active ingredients under acidic conditions is particularly relevant for immediate release formulations because the stomach has a pH of 1.5 to 3.5.

**Table 4B Dissolution Media: pH 6.8: Phosphate Buffer, paddle, 50 rpm, 1000mL**

| | Example 3 | | Example 5 | |
|---|---|---|---|---|
| minutes | Metformin | Vildagliptin | Metformin | Vildagliptin |
| 15 | 60 | 50 | 78 | 73 |
| 30 | 98 | 94 | 99 | 99 |

In the examples according to the disclosure, 50% of the active ingredients are dissolved at 15 minutes and over 90% are dissolved at 30 minutes in neutral conditions. From the above two tables it can be seen that formulations according to Example 5 have a higher dissolution rate than those of Example 3 under both sets of conditions. The addition of extragranular microcrystalline cellulose and sodium stearyl fumarate to the second portion has therefore been surprisingly found to shorten the dissolution times.

### Comparative example

Table 5: Impurity content after 14 days at 50°C for film coated tablets with example 1 formula and Eucreas^{®} (batch WP349). Eucreas^{®} film coated tablets were manufactured according to EP1948149B1. The film coated tablets were prepared using example 6 and 7 process. Water content was determined using Karl-Fischer titration.

| | Ex. N°1 | Eucreas^{®} |
|---|---|---|
| Total impurity content increase (w/w %) | 0.37 | 0.61 |

From the above table, it can be seen that after 14 days at 50°C, tablets prepared according to Example 1 show a lower increase in total impurity content than Eucreas^{®} (batch WP349).

## Claims

1. A pharmaceutical composition in the form of immediate release tablets comprising at least a granulated portion which was granulated prior to its incorporation into a tablet, and
at least a second portion,
wherein the granulated portion comprises metformin or a pharmaceutically acceptable salt thereof, an amount of water below 5 wt%, relative to the weight of the granulated portion, and optionally a binder, and
wherein the second portion comprises
vildagliptin or a pharmaceutically acceptable salt thereof,
wherein vildagliptin is crystalline vildagliptin, and
wherein vildagliptin is not in the form of granules,
a diluent, wherein the diluent is a mixture of lactose and microcrystalline cellulose
wherein the lactose is present in an amount of from 1 to 15%, or further preferably from 7 to 9% by weight, in respect of the total amount of the pharmaceutical composition and
wherein the microcrystalline cellulose is preferably present in an amount of from 1 to 5%, or further preferably from 1 to 3% by weight, in respect of the total amount of the pharmaceutical composition,
a disintegrating agent, and
a lubricant,
wherein the lubricant is selected from the group comprising magnesium stearate and sodium stearyl fumarate.

2. The pharmaceutical composition according to any one of the preceding claims wherein the granulated portion comprises an amount of water below from 0.1 to 4 wt %, preferably 0.3 to 3 wt % and further preferably from 0.4 to 1.4 wt % relative to the weight of the granulated portion.

3. The pharmaceutical composition according to any one of the preceding claims wherein vildagliptin is crystalline vildagliptin form A **characterised by** X-Ray diffraction peaks at 12.0, 13.5, 16.6, 17.1, 17.2, 20.1, 22.5, 27.4 and 28.1 ± 0.2 degrees 2-theta.

4. The pharmaceutical composition according to any one of the preceding claims, wherein the binder is a monosaccharide, a disaccharide or a polysaccharide, or a mixture of more than one thereof, preferably it is a cellulose derivative, more preferably the binder is hydroxypropyl cellulose.

5. The pharmaceutical composition according to any one of the preceding claims, wherein the lubricant is sodium stearyl fumarate.

6. The pharmaceutical composition according to any one of the preceding claims, wherein the disintegrant is selected from a group comprising crospovidone, croscarmellose sodium, and is preferably croscarmellose sodium.

7. The pharmaceutical composition according to any one of the preceding claims, wherein the total amount of metformin is from 60 to 90%, preferably from 65 to 79% in respect of the total amount of the pharmaceutical composition.

8. The pharmaceutical composition according to any one of the preceding claims, wherein the total amount of diluent is from 2 to 16 %, preferably from 5 to 15 % and still preferably from 8 to 12% in respect of the total amount of the pharmaceutical composition.

9. The pharmaceutical composition according to any one of the preceding claims, wherein the binder is present in an amount from 1 to 10%, preferably from 3 to 7% by weight of the binder, in respect of the total amount of the pharmaceutical composition.

10. The pharmaceutical composition according to any one of the preceding claims, wherein the lubricant present in an amount from 0.1 to 5%, or further preferably from 0.5 to 2% by weight, in respect of the total amount of the pharmaceutical composition.

11. The pharmaceutical composition according to any one of the preceding claims, wherein the disintegrant is present in an amount of from 0.1 to 10%, or further preferably from 0.2 to 5% by weight, preferably from 0.8 to 3% in respect of the total amount of the pharmaceutical composition.

12. A process for the manufacture of the pharmaceutical composition as defined in claims 1 to 11 wherein the process comprises the following steps:
a) Granulating the metformin, and optionally a binder, to form a granulated portion;
b) Blending the granulated portion with vildagliptin or salts thereof, the diluent, a disintegrant and a lubricant to obtain a final blend;
c) Compressing the final blend into tablets;
d) Optionally, applying a coating on the tablets obtained in step c).

13. The process according to claim 12, wherein the granulation is wet granulation, preferably where the solvent is water, and optionally wherein the binder is selected from acacia, tragacanth, sucrose, sodium alginate, methyl cellulose, hydroxypropyl cellulose, pyrrolidone and ethyl cellulose.

## Patentansprüche

1. Pharmazeutische Zusammensetzung in Form von Tabletten mit sofortiger Wirkstofffreisetzung, umfassend mindestens einen granulierten Teil, der vor seiner Einarbeitung in eine Tablette granuliert wurde,
und
mindestens einen zweiten Teil,
wobei der granulierte Teil Metformin oder ein pharmazeutisch annehmbares Salz davon, eine Wassermenge von unter 5 Gew.-%, bezogen auf das Gewicht des granulierten Teils, und gegebenenfalls ein Bindemittel umfasst, und
wobei der zweite Teil Folgendes umfasst:
Vildagliptin oder ein pharmazeutisch annehmbares Salz davon,
wobei das Vildagliptin kristallines Vildagliptin ist, und
wobei das Vildagliptin nicht in Form von Granulat vorliegt,
ein Verdünnungsmittel, wobei das Verdünnungsmittel eine Mischung aus Lactose und mikrokristalliner Cellulose ist,
wobei die Lactose in einer Menge von 1 bis 15 % oder weiter bevorzugt von 7 bis 9 Gew.-%, bezogen auf die Gesamtmenge der pharmazeutischen Zusammensetzung, vorliegt und
wobei die mikrokristalline Cellulose vorzugsweise in einer Menge von 1 bis 5 % oder weiter bevorzugt von 1 bis 3 Gew.-%, bezogen auf die Gesamtmenge der pharmazeutischen Zusammensetzung, vorliegt,
ein Sprengmittel, und
ein Schmiermittel,
wobei das Schmiermittel ausgewählt ist aus der Gruppe umfassend Magnesiumstearat und Natriumstearylfumarat.

2. Pharmazeutische Zusammensetzung nach einem der vorhergehenden Ansprüche, wobei der granulierte Teil eine Wassermenge von 0,1 bis 4 Gew.-%, vorzugsweise von 0,3 bis 3 Gew.-% und weiter bevorzugt von 0,4 bis 1,4 Gew.-%, bezogen auf das Gewicht des granulierten Teils, umfasst.

3. Pharmazeutische Zusammensetzung nach einem der vorhergehenden Ansprüche, wobei das Vildagliptin kristallines Vildagliptin der Form A ist, **gekennzeichnet durch** Röntgenbeugungspeaks bei 12,0, 13,5, 16,6, 17,1, 17,2, 20,1, 22,5, 27,4 und 28,1 ± 0,2 Grad 2-Theta.

4. Pharmazeutische Zusammensetzung nach einem der vorhergehenden Ansprüche, wobei das Bindemittel ein Monosaccharid, ein Disaccharid oder ein Polysaccharid oder eine Mischung aus mehreren davon ist, wobei es vorzugsweise ein Cellulosederivat ist, wobei das Bindemittel stärker bevorzugt Hydroxypropylcellulose ist.

5. The pharmaceutical composition according to any one of the preceding claims, wherein the lubricant is sodium stearyl fumarate.

6. Pharmazeutische Zusammensetzung nach einem der vorhergehenden Ansprüche, wobei das Sprengmittel ausgewählt ist aus einer Gruppe umfassend Crospovidon und Croscarmellose-Natrium, und vorzugsweise Croscarmellose-Natrium ist.

7. Pharmazeutische Zusammensetzung nach einem der vorhergehenden Ansprüche, wobei die Gesamtmenge an Metformin 60 bis 90 %, vorzugsweise 65 bis 79 %, bezogen auf die Gesamtmenge der pharmazeutischen Zusammensetzung, beträgt.

8. Pharmazeutische Zusammensetzung nach einem der vorhergehenden Ansprüche, wobei die Gesamtmenge an Verdünnungsmittel 2 bis 16 %, vorzugsweise 5 bis 15 % und noch bevorzugter 8 bis 12 %, bezogen auf die Gesamtmenge der pharmazeutischen Zusammensetzung, beträgt.

9. Pharmazeutische Zusammensetzung nach einem der vorhergehenden Ansprüche, wobei das Bindemittel in einer Menge von 1 bis 10 Gew.-%, vorzugsweise von 3 bis 7 Gew.-% des Bindemittels, bezogen auf die Gesamtmenge der pharmazeutischen Zusammensetzung, vorliegt.

10. Pharmazeutische Zusammensetzung nach einem der vorhergehenden Ansprüche, wobei das Schmiermittel in einer Menge von 0,1 bis 5 Gew.-% oder weiter bevorzugt von 0,5 bis 2 Gew.-%, bezogen auf die Gesamtmenge der pharmazeutischen Zusammensetzung, vorliegt.

11. Pharmazeutische Zusammensetzung nach einem der vorhergehenden Ansprüche, wobei das Sprengmittel in einer Menge von 0,1 bis 10 Gew.-% oder weiter bevorzugt von 0,2 bis 5 Gew.-%, vorzugsweise von 0,8 bis 3 Gew.-%, bezogen auf die Gesamtmenge der pharmazeutischen Zusammensetzung, vorliegt.

12. Verfahren zur Herstellung der pharmazeutischen Zusammensetzung wie in den Ansprüchen 1 bis 11 definiert, wobei das Verfahren die folgenden Schritte umfasst:
a) Granulieren des Metformins und gegebenenfalls eines Bindemittels zur Bildung eines granulierten Teils;
b) Mischen des granulierten Teils mit Vildagliptin oder Salzen davon, dem Verdünnungsmittel, einem Sprengmittel und einem Schmiermittel zum Erhalt einer Endmischung;
c) Verpressen der Endmischung zu Tabletten;
d) Gegebenenfalls Aufbringen eines Überzugs auf die in Schritt c) erhaltenen Tabletten.

13. Verfahren nach Anspruch 12, wobei die Granulierung eine Feuchtgranulierung ist, vorzugsweise wobei das Lösungsmittel Wasser ist, und wobei das Bindemittel gegebenenfalls ausgewählt ist aus Gummi arabicum, Tragant, Saccharose, Natriumalginat, Methylcellulose, Hydroxypropylcellulose, Pyrrolidon und Ethylcellulose.

## Revendications

1. Composition pharmaceutique sous la forme de comprimés à libération immédiate comprenant au moins une portion granulée qui a été granulée préalablement à son incorporation dans un comprimé,
et
au moins une seconde portion,
dans laquelle la portion granulée comprend de la metformine ou l'un de ses sels pharmaceutiquement acceptables, une quantité d'eau inférieure à 5 % en poids, par rapport au poids de la portion granulée, et éventuellement un liant, et
dans laquelle la seconde portion comprend
de la vildagliptine ou l'un de ses sels pharmaceutiquement acceptables,
ladite vildagliptine étant de la vildagliptine cristalline, et
ladite vildagliptine n'étant pas sous la forme de granulés,
un diluant, ledit diluant étant un mélange de lactose et de cellulose microcristalline
dans laquelle le lactose est présent en une quantité allant de 1 à 15 %, ou de manière davantage préférée de 7 à 9 % en poids, par rapport au poids total de la composition pharmaceutique et
dans laquelle la cellulose microcristalline est de préférence présente en une quantité allant de 1 à 5 %, ou de manière davantage préférée de 1 à 3 % en poids, par rapport au poids total de la composition pharmaceutique,
un agent de désintégration, et
un lubrifiant,
dans laquelle le lubrifiant est choisi dans le groupe comprenant le stéarate de magnésium et le fumarate de stéaryle sodique.

2. Composition pharmaceutique selon l'une quelconque des revendications précédentes, dans laquelle la portion granulée comprend une quantité d'eau allant 0,1 à 4 % en poids, de préférence de 0,3 à 3 % en poids et de manière davantage préférée 0,4 à 1,4 % en poids, par rapport au poids de la portion granulée.

3. Composition pharmaceutique selon l'une quelconque des revendications précédentes, dans laquelle la vildagliptine est la vildagliptine cristalline de forme A **caractérisée par** des pics de diffraction des rayons X à 12,0, 13,5, 16,6, 17,1, 17,2, 20,1, 22,5, 27,4 et 28,1 ± 0,2 degrés 2-thêta.

4. Composition pharmaceutique selon l'une quelconque des revendications précédentes, dans laquelle le liant est un monosaccharide, un disaccharide ou un polysaccharide, ou un mélange de plusieurs de ceux-ci, de préférence est un dérivé de cellulose, de manière davantage préférée le liant est l'hydroxypropylcellulose.

5. Composition pharmaceutique selon l'une quelconque des revendications précédentes, dans laquelle le lubrifiant est le fumarate de stéaryle sodique.

6. Composition pharmaceutique selon l'une quelconque des revendications précédentes, dans laquelle l'agent de désintégration est choisi dans le groupe comprenant la crospovidone et la croscarmellose sodique, et est de préférence la croscarmellose sodique.

7. Composition pharmaceutique selon l'une quelconque des revendications précédentes, dans laquelle la quantité totale de metformine est de 60 à 90 %, de préférence de 65 à 79 %, par rapport au poids total de la composition pharmaceutique.

8. Composition pharmaceutique selon l'une quelconque des revendications précédentes, dans laquelle la quantité totale de diluant est de 2 à 16 %, de préférence de 5 à 15 % et de manière encore préférée de 8 à 12 %, par rapport au poids total de la composition pharmaceutique.

9. Composition pharmaceutique selon l'une quelconque des revendications précédentes, dans laquelle le liant est présent en une quantité de 1 à 10 %, de préférence de 3 à 7 % en poids du liant, par rapport au poids total de la composition pharmaceutique.

10. Composition pharmaceutique selon l'une quelconque des revendications précédentes, dans laquelle le lubrifiant est présent en une quantité de 0,1 à 5 %, ou de manière davantage préférée de 0,5 à 2 % en poids, par rapport au poids total de la composition pharmaceutique.

11. Composition pharmaceutique selon l'une quelconque des revendications précédentes, dans laquelle l'agent de désintégration est présent en une quantité de 0,1 à 10 %, ou de manière davantage préférée de 0,2 à 5 % en poids, de préférence de 0,8 à 3 %, par rapport au poids total de la composition pharmaceutique.

12. Procédé de fabrication de la composition pharmaceutique telle que définie dans les revendications 1 à 11, dans lequel le procédé comprend les étapes suivantes :
a) Granuler la metformine, et éventuellement un liant, pour former une portion granulée ;
b) Mélanger la portion granulée avec de la vildagliptine ou ses sels, le diluant, un agent de désintégration et un lubrifiant pour obtenir un mélange final ;
c) Comprimer le mélange final en comprimés ;
d) Éventuellement, appliquer un enrobage sur les comprimés obtenus à l'étape c).

13. Procédé selon la revendication 12, dans lequel la granulation est une granulation humide, de préférence dans lequel le solvant est l'eau, et éventuellement dans lequel le liant est choisi parmi l'acacia, l'adragante, le saccharose, l'alginate de sodium, la méthylcellulose, l'hydroxypropylcellulose, la pyrrolidone et l'éthylcellulose.
